# EUROPEAN PATENT SPECIFICATION

(11) **EP 2 200 569 B1**
(45) Date of publication and mention of the grant of the patent: **25.05.2016**
(21) Application number: 08834648.1
(22) Date of filing: 25.09.2008
(51) Int. Cl.: A61K 8/04, A61K 9/12, A61L 15/44

(54) **KIT FOR CARE OF FOOT AND/OR HAND**
KIT ZUR PFLEGE VON FÜSSEN UND/ODER HÄNDEN
COFFRET POUR SOIN DES PIEDS ET/OU DES MAINS

(30) Priority: 25.09.2007 NL 1034425
(43) Date of publication of application: 30.06.2010
(73) Proprietor: Bioclin B.V., 2628 XJ Delft (NL)
(72) Inventor: KOUMANS, Floris Jan Robert, NL-2611 XD Delft (NL)
(74) Representative: V.O.
(86) International application number: PCT/NL2008/050619
(87) International publication number: WO 2009/041815

(56) References cited:
- EP-A- 1 671 555
- FR-A- 2 604 869
- NL-C1- 1 027 880
- US-A1- 2001 046 514
- US-A1- 2003 129 180

## Description

The invention relates to a system for care of foot and/or hand.

Taking care of, for instance, the skin of a foot and/or hand is usually done by applying a care product to the foot and/or hand. The care product in such cases is usually in the form of a cream, lotion, powder, gel or spray.

US 2003/0 129 180 describes a method for treating a foot infection, whereby the skin of the foot is treated with an enzyme which may be in the form of a foam.

EP 1 671 555 describes a work glove which is provided on the outside with a foam layer of, for instance, rubber or vinyl chloride. The foam layer is an integral part of the work glove and affords the work glove a better grip on the outside.

US 2001/0 046 514 describes a sock of a woven material, with a medicinal product incorporated in the fabric of the sock. This sock is worn under an ordinary sock and is for single use. A drawback of such a sock is that the medicinal product in powder form cannot spread over the entire surface of the foot, for instance between the toes.

FR 2 604 869 describes a glove which is provided on the outside with microcapsules containing a medicinal or care substance. The glove is used to apply the medicinal or care substance to sites of the body without the medicinal or care substance coming into contact with the skin of the hands. A drawback of such a glove is that it is difficult to apply the care product to sites which are difficult to reach, such as the foot. Furthermore, with such a glove it is difficult to apply a care product to the hands properly.

NL 1 027 880 describes a holder with a foam product, which holder is provided with a tube allowing the foam product to be introduced into the pharynx.

A drawback of the use of a care product on a foot and/or hand is that it may be difficult to apply the care product especially to the foot, for instance because the user's capacity to move may be limited for instance due to old age, obesity or disease. It may also be difficult for the user to apply the care product to the hands, for instance due to stiff hands. During the time of action of the product on the foot and/or hand, the user cannot make optimum use of his foot and/or hand.

Another drawback of the use of a care product is that the contact time and contact intensity of the product with the foot and/or the hand strongly depend on the manner in which the product is applied to the foot and/or the hand.

Object of the invention is to provide a system for care of foot and/or hand which obviates the above-mentioned disadvantages.

To this end, the invention provides a system for care of foot and/or hand comprising a wet foam product for taking care of foot and/or hand and a flexible, moisture-proof sleeve for surrounding foot and/or hand in cooperation with the foam product. The system is preferably in two parts and in that case comprises a holder with a foam product for taking care of foot and/or hand and a set of one or more flexible, moisture-proof sleeves.

By making use of a system of a wet foam product and a flexible, moisture-proof sleeve, the foot and/or hand with the foam product can be surrounded by the flexible sleeve, so that the user can use his foot and/or hand while the foam product can act on the skin of the foot and/or hand.

By using a care product in the form of a wet foam product, the foam easily spreads, for instance between the toes or the fingers, so that a relatively large contact surface with the foam product can be obtained. Since during the product's action the foot and/or hand is surrounded by a flexible, moisture-proof sleeve, the wet foam product can remain on the foot and/or hand, so that the action of the foam product can be improved. The user does not need to wait until the time of action of the care product has elapsed before using his foot and/or hand, since his foot and/or hand is surrounded by the flexible, moisture-proof sleeve. Both the duration of contact and the contact intensity of the care product can thus be optimized.

By packaging the wet foam product in a holder, it is relatively simple for a user to use the system with the holder on the one hand and a flexible sleeve on the other. The holder may for instance be a bottle, a dispenser or an aerosol container. Preferably, the wet foam product is packaged in the holder while liquid. The user can simply operate the holder and by hand apply foam product to the foot and/or hand and then surround the foot and/or hand with the flexible sleeve or he can apply the foam product in the sleeve which surrounds the foot and/or hand.

The sleeve preferably remains on the foot and/or hand during the time of action of the foam product. As the sleeve is of flexible design, movement and/or use of the hand and/or foot surrounded with the sleeve is possible. Soiling of the environment by the foaming product applied to the hand and/or foot during use of the hand and/or foot can thus be obviated.

The foam product can for instance have a care and/or a fungicidal and/or an antibacterial action. The foam product is a wet foam. In the holder, the foam product is liquid; as soon as the foam product is out of the holder, it forms a foaming product. The foaming product may for instance dry on the foot and/or hand after a time of action or may be left on the foot and/or hand as a wet film after a time of action.

The flexible, moisture-proof sleeve may be designed to have a single opening which is provided with a cuff edge for fitting around a wrist of a hand and/or an ankle of a foot. The cuff edge of the opening of the sleeve may be designed as an elastic edge, or may be provided, for instance, with a string or Velcro allowing the cuff edge of the sleeve to be fitted around the hand or foot. Also, the cuff edge may form an insertion opening, allowing the sleeve to be used like a sock or mitten. Preferably, the cuff edge of the opening of the sleeve, during use, fits relatively closely around a wrist of a hand or an ankle of a foot. This can prevent moisture escaping from the sleeve during use of the sleeve. Also, since the sleeve is connectible around the wrist and/or ankle, a substantially closed-off environment can be created in which the foam product can act on the foot and/or hand relatively optimally.

By virtue of the cuff edge, the flexible sleeve can be arranged over the foot and/or hand relatively easily. Preferably, the cuff edge is of such design that during arrangement of the sleeve, the opening is sufficiently large for the foot and/or hand to be inserted therein. Thereupon, when the sleeve surrounds the foot and/or hand, the cuff edge can fit relatively closely around the ankle of the foot and/or the wrist of the hand.

The flexible, moisture-proof sleeve is preferably designed in plastic, such as for instance polyethylene, polyester or polyvinyl chloride. For instance, the sleeve may be designed as a flexible, plastic little bag having an opening surrounded with an elastic cuff edge.

US '180 describes that a sock or shoe can be treated separately with a particular type of foam; however, this is to disinfect the sock or shoe itself, hence not the foot.

The invention also relates to a method of taking care of foot and/or hand, wherein a wet foam product is applied to the foot and/or hand and thereupon a flexible, moisture-proof sleeve is arranged, thereby enclosing the foam product applied, around the foot and/or hand for cooperation with the wet foam product.

The invention furthermore relates to the use of a flexible, moisture-proof sleeve for surrounding a foot and/or hand in cooperation with a wet foam product for taking care of foot and/or hand.

The invention furthermore relates to a kit for care of foot and/or hand, comprising a holder with foam product for taking care of foot and/or hand and a set of one or more flexible, moisture-proof sleeves for surrounding foot and/or hand in cooperation with the foam product.

Further advantageous embodiments of the invention are set forth in the subclaims.

The invention will now be elucidated on the basis of an exemplary embodiment which is represented in a drawing. In the drawing:
Fig. 1 schematically shows a system for care of foot and/or hand according to the invention; and
Fig. 2 schematically shows a foot surrounded by a sleeve and foam product of Fig. 1.

The figures are only schematic representations of a preferred embodiment of the invention, and are given by way of non-limiting exemplary embodiment. In the figures, equal parts are represented with equal reference numerals.

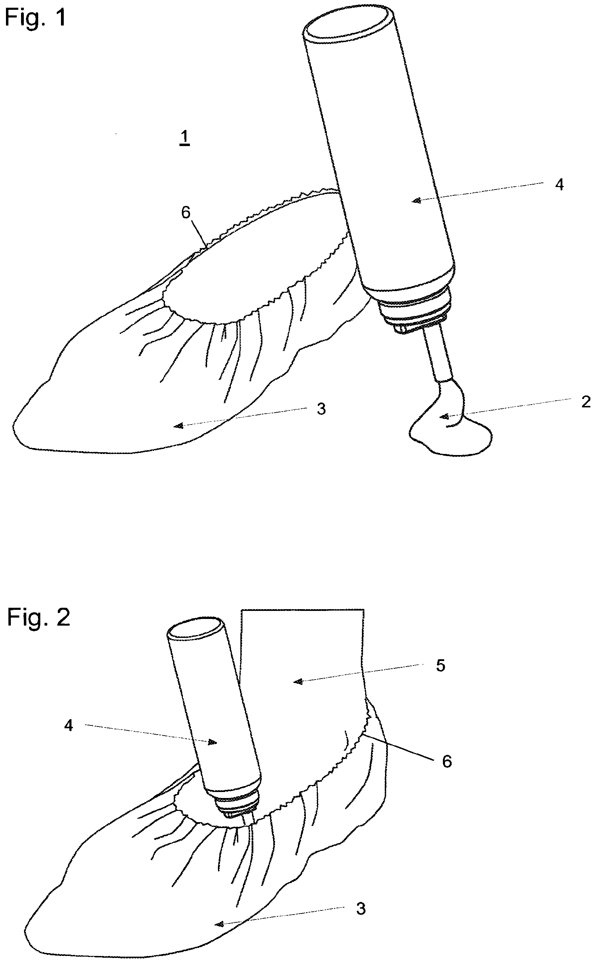

A system for care of foot and/or hand is schematically represented in Fig. 1. Fig. 1 shows a system 1 which is in two parts, one part of the system 1 being formed by a holder 4 with a wet, liquid foam product 2, and a second part of the system 1 being formed by a flexible, moisture-proof sleeve 3. Also, the second part of the system 1 may be formed by a set of flexible, moisture-proof sleeves 3.

The wet foam product 2 in this exemplary embodiment is packaged in a foam dispenser 4. The foam product 2 is usually packaged as a liquid substance and in that case comes out of the package as a wet foam. Foam has a large volume and spreads relatively easily over the skin surface. A drop of still liquid foam can generate a relatively large amount of foam, so that a relatively small package is adequate for many moments of care.

By making use of a care product in the form of a foam, relatively little liquid foam product allows a relatively large volume of foaming product to be created, so that the use of the product can be relatively efficient. Also, making use of foam allows a higher concentration of active substance to be used and/or allows working with relatively more expensive basic constituents while the costs for the product can yet remain advantageous. Also, the duration of contact and/or intensity of contact with the foam product can be optimized.

The flexible, moisture-proof sleeve 3 is arranged on the foot and/or hand for cooperation with the wet foam product 2. The time of action of the wet foam product 2 on the foot and/or hand is relatively short, for instance a few minutes to an hour. During the time of action of the foam product 2, the sleeve 3 is preferably worn independently on the foot and/or hand, without a sock or glove also being worn on the foot and/or hand. After the time of action, the sleeve 3 may be removed from the foot and/or hand. Owing to the flexibility of the sleeve 3, movement of the foot and/or hand during the time of action may be possible and comfort during use is enhanced.

Fig. 2 shows a foot 5 around which a sleeve 3 in the form of a foot sleeve has been arranged, which cooperates with the foam product 2 on the skin of the foot 5. The sleeve 3 is loose-fitting and is provided with a single opening which is provided with a cuff edge 6. During use of the sleeve 3 the cuff edge 6 can fit relatively closely around the wrist of a hand or an ankle of a foot. In this exemplary embodiment, the cuff edge 6 is designed as an elastic edge, by which it can enclose the foot. Thus, the sleeve 3 can form a moisture-proof, closed-off environment in which the foaming product can act on the foot relatively optimally. The sleeve 3 may also be in the form of a sock for the foot, or in the form of a glove or mitten for the hand. In case of a sleeve 3 designed as a sock for a foot, the cuff edge 6 can enclose an insert opening for the foot, while the cuff edge 6 can fit relatively closely around a leg of the user. Preferably, the sleeve 3 is a simple sleeve, like a mitten or a foot sleeve. The simple sleeve has no branches.

By making use of a flexible, moisture-proof sleeve 3, the foam product 2 can be in contact with the skin surface of, for instance, the foot and/or hand for a longer time, without the user experiencing much hindrance from it. As the sleeve 3 is flexible and moisture-proof, the foam product 2 remains in the sleeve 3. The foaming product will hardly evaporate or run out of the sleeve 3, so that a relatively optimal action of the foaming product on the foot can be obtained. During the time of action of the foam product 2 on the foot, the user can simply walk about as usual, or during action on the hand simply use his hand as usual, and no longer needs to wait for elapse of the time of action. Also, the user will hardly have the problem that the feet and/or hands which are wet, sticky or slippery from a care product might therewith soil things he would touch in the meantime, such as for instance clothing or bedding, since the feet and/or hands are protected by the sleeve 3.

The foam product 2 may be applied from the holder or package 4 directly onto the foot and/or hand, but may also be applied directly into the flexible, moisture-proof sleeve 3. It is also possible first to arrange the flexible, moisture-proof sleeve 3 on the foot and/or the hand, then to spray the foam product 2 into it and then to close off the opening of the sleeve 3 around the foot and/or hand by means of the cuff edge 6.

If the foam product 2 is applied from the holder or package 4 directly onto, for instance, the foot, the user does not need to put care product on his foot anymore. The foam can spread over the skin surface of the foot relatively easily, but can also find its way to sites that are relatively difficult to reach, as between the toes, so that the contact surface with the active constituents from the foam can be relatively large. This is advantageous when the user can reach his feet only with difficulty, for instance due to old age, disease or obesity. The user can then put on the flexible, moisture-proof sleeve 3 to surround the foot.

If the user first applies the foam product 2 into the sleeve 3, and then puts the sleeve 3 with foam product 2 therein on the foot and/or hand, the operations for the user are simple and comparable to the operations of putting on a shoe or sock. The user then only needs to put on the sleeve 3 as a kind of sock over the foot or to pull it as a kind of mitten over the hand. The foam product 2 in the sleeve 3 spreads over the skin surface and can thus come into contact with the skin to be taken care of. This makes it easy for the user to take care of his feet and/or hands.

Also, the user can first put on the sleeve 3, for instance on the foot, and then spray the foam product 2 into the sleeve 3. The user can for instance keep the sleeve 3 open to form an opening for a nozzle of the foam dispenser 4. The foam product spreads easily over the skin surface. The user experiences hardly any hindrance from the presence of the foam product 2 owing to the sleeve 3, which allows the user to continue using his foot and/or hand during the time of action of the foam product. After the time of action for the foam product 2 has elapsed, the sleeve 3 can be taken off the foot and/or hand. The time of action of the foam product may vary from a few minutes to a couple of hours.

After the time of action, the foam product 2 may have dried on the skin or have become liquid again. Liquefied foam product remains in the sleeve 3 since it is of moisture-proof design. Preferably, the sleeve is manufactured from plastic. Optionally, a thin film of the foam product may remain behind on the skin, which can be dried up or can dry in air.

After use, the user can rinse the sleeve 3 for reuse or throw it away. The system 1 can also comprise a set of a foam product 2 with a number of flexible, moisture-proof sleeves 3. These may for instance be different kinds of sleeves 3, such as foot sleeve or sock for the foot or glove or mitten for the hand. Also, these may be several sleeves 3 of a same kind, so that the user can elect to throw away a sleeve 3 after use.

Other designs are also possible. For instance, the package for the wet, liquid foam product may be designed as an aerosol bottle or a pump foamer. The aerosol bottle may be provided with a spout at the spray opening, allowing the foam product to be simply sprayed into the sleeve, also when the sleeve is already disposed on the foot and/or hand. Also, the foam product may not only be directed to care of the skin of foot and/or hand, but may also be directed to the prevention of skin problems such as fungus, infection, callosity, or for instance be directed to tissue restoration.

Such variants will be clear to those skilled in the art and are understood to fall within the scope of the invention as set forth in the following claims.

## Claims

1. A system (1) for care of foot and/or hand wherein the system is in two parts, comprising a wet foam product (2) for taking care of foot and/or hand and a flexible, moisture-proof sleeve (3) for surrounding foot (5) and/or hand in cooperation with the foam product (2), wherein the wet foam product (2) is packaged in a holder (4).

2. A system according to claim 1, comprising a set of a foam product (2) and a number of flexible, moisture-proof sleeves (3).

3. A system according to claim 1 or 2, wherein the sleeve (3) is in the form of a foot sleeve, sock, glove or mitten.

4. A system according to any one of the preceding claims, wherein an opening of the sleeve is provided with a cuff edge (6) for fitting around a wrist of a hand and/or an ankle of a foot.

5. A system according to any one of the preceding claims, wherein the cuff edge (6) of the sleeve is elastic.

6. A system according to any one of the preceding claims, wherein the sleeve (3) is a simple sleeve.

7. A system according to any one of the preceding claims, wherein the sleeve (3) is manufactured from plastic.

8. A system according to any one of the preceding claims, wherein the sleeve (3) is disposable.

9. A kit for taking care of foot and/or hand comprising a holder (4) with a wet foam product (2) for taking care of foot and/or hand and a set of one or more flexible, moisture-proof sleeves (3) for surrounding foot and/or hand in cooperation with the foam product.

## Patentansprüche

1. System (1) zur Pflege von Füßen und/oder Händen, wobei das System aus zwei Teilen besteht, umfassend ein Nassschaumprodukt (2) zum Versorgen von Füßen und/oder Händen und eine flexible, feuchtigkeitsdichte Hülle (3), um die Füße (5) und/oder Hände in Zusammenarbeit mit dem Schaumprodukt (2) zu umgeben, wobei das Nassschaumprodukt (2) in einen Halter (4) verpackt ist.

2. System nach Anspruch 1, umfassend ein Set eines Schaumprodukts (2) und eine Anzahl von flexiblen, feuchtigkeitsdichten Hüllen (3).

3. System nach Anspruch 1 oder 2, wobei die Hülle (3) in Form einer Fußhülle, einer Socke, eines Handschuhs oder eines Fäustlings ist.

4. System nach einem der vorhergehenden Ansprüche, wobei eine Öffnung der Hülle mit einem Stulpenrand (6) versehen ist, sodass sie um ein Gelenk der Hand und/oder einen Knöchel des Fußes passt.

5. System nach einem der vorhergehenden Ansprüche, wobei der Stulpenrand (6) der Hülle elastisch ist.

6. System nach einem der vorhergehenden Ansprüche, wobei die Hülle (3) eine einfache Hülle ist.

7. System nach einem der vorhergehenden Ansprüche, wobei die Hülle (3) aus Kunststoff hergestellt ist.

8. System nach einem der vorhergehenden Ansprüche, wobei die Hülle (3) eine Wegwerfhülle ist.

9. Kit zur Pflege von Füßen und/oder Händen, umfassend einen Halter (4) mit einem Nassschaumprodukt (2) zum Versorgen von Füßen und/oder Händen und ein Set von einer oder mehreren flexiblen, feuchtigkeitsdichten Hüllen (3), um die Füße und/oder Hände in Zusammenarbeit mit dem Schaumprodukt zu umgeben.

## Revendications

1. Système (1) de soin des pieds et/ou des mains, dans lequel le système est en deux parties, qui comprend un produit moussant humide (2) destiné à prendre soin des pieds et/ou des mains, et une enveloppe de protection flexible et résistante à l'humidité (3) destinée à entourer les pieds (5) et/ou les mains en coopération avec le produit moussant (2), dans lequel le produit moussant humide (2) est contenu dans un récipient (4).

2. Système selon la revendication 1, qui comprend un kit composé d'un produit moussant (2) et de plusieurs enveloppes flexibles et résistantes à l'humidité (3).

3. Système selon la revendication 1 ou 2, dans lequel l'enveloppe (3) se présente sous la forme d'un chausson, d'une chaussette, d'un gant ou d'une moufle.

4. Système selon l'une quelconque des revendications précédentes, dans lequel une ouverture de l'enveloppe est munie d'un rebord (6) destiné à être placé autour d'un poignet d'une main et/ou de la cheville d'un pied.

5. Système selon l'une quelconque des revendications précédentes, dans lequel le rebord (6) de l'enveloppe est élastique.

6. Système selon l'une quelconque des revendications précédentes, dans lequel l'enveloppe (3) est une enveloppe simple.

7. Système selon l'une quelconque des revendications précédentes, dans lequel l'enveloppe (3) est fabriquée en plastique.

8. Système selon l'une quelconque des revendications précédentes, dans lequel l'enveloppe (3) est jetable.

9. Kit de soin des pieds et/ou des mains qui comprend un récipient (4) avec un produit moussant humide (2) destiné à prendre soin des pieds et/ou des mains, et un kit composé d'une ou plusieurs enveloppe(s) flexible(s) et résistante(s) à l'humidité (3) destinée(s) à entourer les pieds et/ou les mains en coopération avec le produit moussant.
